Europäisches Patentamt

European Patent Office

Office européen des brevets

(11) Veröffentlichungsnummer: **0 452 728 A1**

# (12) EUROPÄISCHE PATENTANMELDUNG

(21) Anmeldenummer: 91105082.1

(22) Anmeldetag: 28.03.91

(51) Int. Cl.⁵: **B05B 11/02**, A61M 11/00

(30) Priorität: 17.04.90 DE 4012270
18.05.90 DE 4016126

(43) Veröffentlichungstag der Anmeldung:
23.10.91 Patentblatt 91/43

(84) Benannte Vertragsstaaten:
CH DE FR GB IT LI

(71) Anmelder: COSTER TECNOLOGIE SPECIALI S.P.A.
Via Fabio Filzi 27
I-20124 Mailand(IT)

(72) Erfinder: Geier, Adalbert
Via Valcolgher
I-38050 Villazzano, Trient(IT)

(74) Vertreter: Popp, Eugen, Dr. et al
MEISSNER, BOLTE & PARTNER Postfach 86 06 24
W-8000 München 86(DE)

(54) **Vorrichtung zur transnasalen oder oralen Verabreichung von Medikamenten oder dgl.**

(57) Vorrichtung zur transnasalen oder oralen Verabreichung von Medikamenten oder dgl. Medien, mit einem fingerartigen Applikator (3), an dessen freiem Ende eine Zerstäuberdüse (6, 10) angeordnet ist und der einen Zylinderraum (1 ) zur Aufnahme des zu verabreichenden Mediums umfaßt, wobei dieses mittels eines Kolbens (7) aus dem Zylinderraum (1) über die Zerstäuberdüse (6) verabreichbar ist. Der Zerstäuberdüse (6) ist eine membranartige Dichtung (4) mit einem sich nur unter Druck öffnenden Fluiddurchgang vorgeordnet.

FIG. 1

Die Erfindung betrifft eine Vorrichtung zur transnasalen oder oralen Verabreichung von Medikamenten oder dgl. Medien, gemäß dem Oberbegriff des Anspruches 1.

Eine derartige Vorrichtung ist bekannt. Sie wurde entwickelt unter dem Gesichtspunkt, daß die Nasenschleimhaut oder auch der Rachen ein ideales Applikationsfeld für Medikamente sind. An die Technik derartiger Vorrichtungen stellt man dabei höchste Anforderungen. Denn die Dosiergenauigkeit entscheidet, welche Medikamente in dieser Form angewendet werden können. Darüber hinaus ist auch von Bedeutung, daß die Handhabung anwenderfreundlich ist. Diese beiden Voraussetzungen müssen erfüllt sein, um eine ausreichend hohe Funktionssicherheit zu gewährleisten, die für die Applikation hochwirksamer Medikamente unbedingt erforderlich ist.

Ein Problem stellt bei den bekannten Vorrichtung die Kontaminationsgefahr dar. Es sind keine vorrichtungs-integrierten Maßnahmen getroffen dafür, daß die Füllung nicht mit Luft und damit mit der Umgebung in Kontakt kommt.
Auch besteht grundsätzlich die Gefahr von Leckagen bei unsachgemäßem Verschluß der Zerstäuberdüse. Schließlich besteht die bekannte Vorrichtung aus relativ viel Einzelbauteilen mit der Folge eines entsprechend hohen Herstellungs-, Material- und Montageaufwandes. Auch wird dadurch die mechanische Funktionssicherheit in Frage gestellt.

Der vorliegenden Erfindung liegt die Aufgabe zugrunde, eine Vorrichtung der eingangs genannten Art zu schaffen, die einfach im Aufbau und dennoch höchst funktionssicher sowie sicher gegen Kontamination ist.

Diese Aufgabe wird erfindungsgemäß durch die kennzeichnenden Merkmale des Anspruches 1 gelöst, wobei die Unteransprüche vorteilhafte Weiterbildungen des Erfindungsgedankens betreffen.

Mit den Maßnahmen nach den Ansprüchen 9 bis 13 ist eine mehrmalige Applikation mit einer vorbestimmten Dosis einer mit Medikamenten versetzten Flüssigkeit möglich. Der vorliegende Erfindungsgedanke bezieht sich also sowohl auf einen Einmalals auch Mehrmal-Dosierer.

Durch die erfindungsgemäß vorgesehene membranartige Dichtung zwischen Zerstäuberdüse und dem das zu verabreichende Medium enthaltenden Pumpen- bzw. Zylinderraum ist ein sicherer Abschluß der mit Medikamenten versetzten Füllung gegenüber der Umgebung dauerhaft gewährleistet. Nur zum Zwecke der Befüllung und/oder der Verabreichung soll die membranartige Dichtung öffnen und einen Fluiddurchgang freigeben bzw. bilden.

Die vorgenannte Membrandichtung ist ein vorrichtungs-integriertes Bauteil, welches nach Montage nicht mehr zugänglich und dementsprechend auch nicht manipulierbar ist. Vorzugsweise ist die Membrandichtung in einem Durchgang zwischen Zylinderraum und Zerstäuberdüse angeordnet, insbesondere unter Klemmfixierung längs ihres Umfangsrandes. Die Klemmfixierung erfolgt nach einem Ausführungsbeispiel durch ein zwischen der Membrandichtung und der Zerstäuberdüse angeordnetes, gleichzeitig zur Verteilung des zu verabreichenden Mediums vor dessen Austritt durch die Zerstäuberdüse dienendes Zwischenstück, wobei an der der Zerstäuberdüse zugewandten Seite des Zwischenstücks vorzugsweise noch eine Einrichtung zur Verwirbelung des durch die Zerstäuberdüse austretenden Mediums angeordnet ist.

Zum Zwecke der Ausbildung eines Fluiddurchgangs durch die Membrandichtung hindurch weist die Membrandichtung einen geraden, sternförmigen oder Kreuz-Schlitz auf, der unter einem vorbestimmten Druck öffnet, und zwar beim Applizieren nach außen hin und beim Befüllen der Vorrichtung nach innen, d. h. in Richtung zum Pumpen- bzw. Zylinderraum hin, der zur Aufnahme des zu verabreichenden Mediums dient.

Der dem vorerwähnten Zylinderraum zugeordnete Kolben wird in Ausgangsstellung vorzugsweise durch einen Rastmechanismus gehalten, der zur Verabreichung durch Ausübung von Druck auf eine an dem der Zerstäuberdüse abgewandten Ende des Kolbens ausgebildete Kolbenbetätigungsfläche überwindbar ist. Die konkrete Ausbildung der Kolbenbetätigungsfläche sowie des Rastmechanismus ist in den Ansprüchen 6 bis 8 beschrieben.

Wie bereits oben angedeutet, ist eine Ausführungsform der erfindungsgemäß ausgebildeten Vorrichtung dadurch gekennzeichnet, daß der Kolben schrittweise in den Zylinderraum unter entsprechender Mehrfach-Verabreichung hineinbewegbar ist. Die konstruktiven Maßnahmen zur Erzielung dieses Effektes sind in den Ansprüchen 10 bis 13 näher beschrieben.

Die Handhabung der erfindungsgemäßen Vorrichtung wird durch die Konstruktionsmerkmale gemäß Anspruch 14 erleichtert.

Die Ausführungsform nach den Ansprüchen 16 und 17 zeichnet sich durch eine Füllpatrone als vom Applikationsmechanismus getrenntes Bauteil aus, so daß letzterer mehrfach verwendbar ist.

Nachstehend werden zwei Ausführungsformen einer erfindungsgemäß ausgebildeten Vorrichtung anhand der beigefügten Zeichnungen näher erläutert. Es zeigen:

Fig. 1        eine Vorrichtung gemäß Erfindung im Längsschnitt;

Fig. 2        den Zerstäuberteil der Vorrichtung gemäß Fig. 1 im Längsschnitt und vergrößertem Maßstab unter Darstellung der erfindungsgemäß vorgesehenen Membrandichtung während der Applikation;

Fig.3    den Zerstäuberteil entsprechend Fig. 2 im Längsschnitt und vergrößertem Maßstab unter Darstellung der erfindungsgemäß vorgesehenen Membrandichtung beim Befüllen der Vorrichtung;

Fig. 4    drei verschiedene Ausführungsformen der erfindungsgemäß verwendeten Membrandichtung in Draufsicht;

Fig. 5    das Detail V in Fig. 1 in vergrößertem Maßstab;

Fig. 6    eine zweite Ausführungsform einer erfindungsgemäß ausgebildeten Vorrichtung in Seitenansicht;

Fig. 7    Vorrichtung gemäß Fig. 6 im Längsschnitt, wobei gegenüber Fig. 6 der Verabreichungskolben sich noch in Ausgangsstellung befindet;

Fig. 8    eine dritte Ausführungsform einer erfindungsgemäßen Vorrichtung im Längsschnitt;

Fig. 9    die zur Vorrichtung gemäß Fig. 8 gehörende Füllpatrone im Längsschnitt;

Fig. 10    eine gegenüber Fig. 9 abgewandelte Füllpatrone, ebenfalls im Längsschnitt;

Fig. 11 bis 14    eine vierte Ausführungsform einer erfindungsgemäßen Vorrichtung entsprechend den Darstellungen in den Fig. 1, 2 und 3 und unter zusätzlicher Darstellung eines bei dieser Ausführungsform verwendeten Glaszylinders (Fig. 12);

Fig. 15    eine Doppel-Applikationsvorrichtung gemäß Erfindung im Längschnitt; und

Fig. 16    eine Abwandlung der Ausführungsform gemäß Fig. 15.

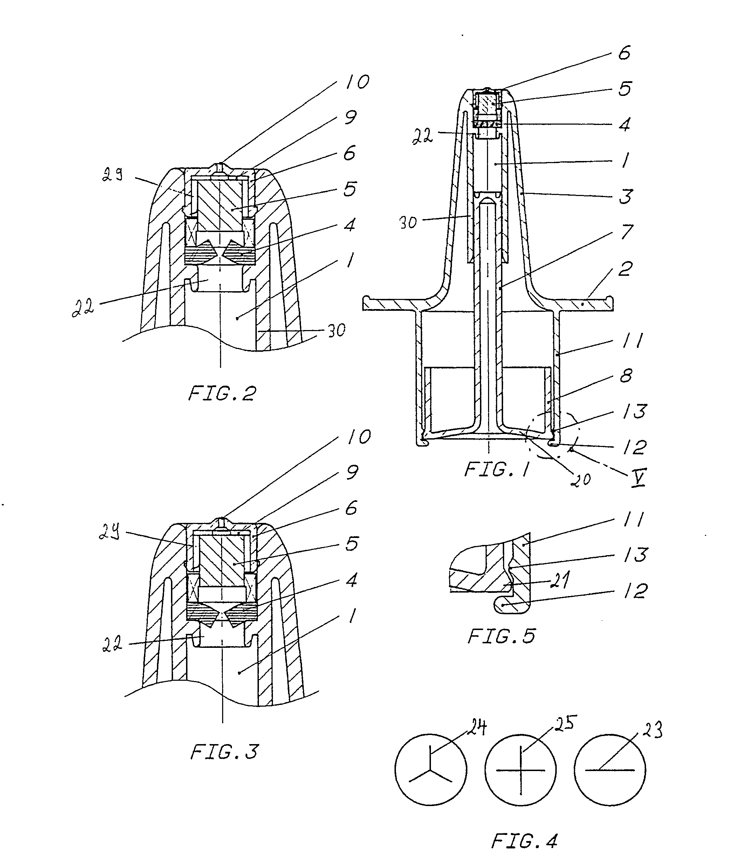

Die in Fig. 1 dargestellte Vorrichtung zur transnasalen oder oralen Verabreichung von Medikamenten oder dgl. Medien, insbesondere flüssigen Medien, weist einen röhrchen- bzw. fingerartigen Applikator 3 auf, an dessen freiem Ende eine Zerstäuberdüse 6 angeordnet ist. Im Innern umfaßt der Applikator 3 einen sich in Applikatorlängsrichtung erstreckenden Zylinderraum 1 zur Aufnahme des zu verabreichenden Mediums, wobei dieses mittels eines Kolbens 7 aus dem Zylinderraum 1 über die Zerstäuberdüse 6 verabreichbar ist. Der Zerstäuberdüse 6 ist eine membranartige Dichtung 4 mit einem sich nur unter Druck öffnenden Fluiddurchgang vorgeordnet. Die Membrandichtung 4 besteht aus einem hochelastischen Material, vorzugsweise Kunststoff, Kautschuk oder Gummi. Auch der Applikator 3 samt Zylinder 1 und Kolben 7 bestehen vorzugsweise aus Kunststoff. Wie die Fig. 2 und 3 besonders gut erkennen lassen, ist die Membrandichtung 4 in einem Durchgang 22 zwischen Zylinderraum 1 und Zerstäuberdüse 6 angeordnet, und zwar längs ihres Umfangsrandes eingeklemmt. Die Klemmfixierung der Membrandichtung 4 erfolgt durch ein zwischen dieser und der Zerstäuberdüse 6 angeordnetes, gleichzeitig zur Verteilung des zu verabreichenden Mediums vor dessen Austritt durch die Zerstäuberdüse 6 dienendes Zwischenstück 5, wobei an der der Zerstäuberdüse 6 zugewandten Seite des Zwischenstücks 5 eine Einrichtung zur Verwirbelung des durch die Zerstäuberdüse 6 austretenden Mediums angeordnet ist. Die genannte Einrichtung wird durch mehrere an der genannten Stelle ausgebildete Wirbelkanäle definiert.

Die vorgenannte Fluidöffnung in der Membrandichtung 4 ist entweder durch einen geraden Schlitz 23, sternförmigen Schlitz 24 oder Kreuzschlitz 25 gebildet. Diese drei Alternativen sind in Fig. 4 jeweils in Draufsicht dargestellt. Im Ausgangszustand ist der Inhalt des Pumpen- bzw. Zylinderraums 1 nach unten durch den Kolben 7 und nach oben durch die Dichtung 4 hermetisch abgeschlossen. Nur durch Betätigung des Kolbens 7 und dem dadurch bewirkten Druckaufbau innerhalb des Zylinderraums 1 wird die Dichtung 4 elastisch nach außen bzw. zur Zerstäuberdüse 6 hin verformt, so daß der vorgenannte Schlitz 23, 24 oder 25 eine Fluidöffnung freigibt. Dieser Zustand ist in Fig. 2 dargestellt. Die Fig. 2 läßt im übrigen noch erkennen, daß die Zerstäuberdüse 6 durch ein topfartiges Bauteil gebildet ist, dessen Boden eine Mikroöffnung 10 aufweist, durch die hindurch das zu verabreichende Medium zerstäubt wird. Zwischen der Innenseite der Seitenwand des vorgenannten topfartigen Zerstäuber-Bauteils und dem Zwischenstück 5 ist ein Ringspalt definiert, durch den hindurch das zu verabreichende Medium sowohl bei der Verabreichung als auch beim Befüllen des Zylinderraums 1 hindurchströmt, und zwar unter Gleichverteilung über den Umfang. Der genannte Ringraum ist in Fig. 2 und 3 jeweils mit der Bezugsziffer 29 gekennzeichnet.

Um den Weg über die Dichtung 4 nach außen freizumachen, muß je nach der Elastizität und Dikke der Dichtung 4 mehr oder weniger Kraft aufgewandt werden. Dies hat einen vorteilhaften Druckaufbau zur Folge, der für eine gute Zerstäubung des zu verabreichenden Mediums durch die Zerstäuberdüse 6 bzw. deren Mikroöffnung 10 hindurch notwendig ist.

In Fig. 3 ist der Zerstäuberteil ähnlich wie in Fig. 2 dargestellt nur mit dem Unterschied, daß Fig. 3 die Verformung der Membrandichtung 4 beim Befüllen des Zylinderraums 1 mit dem zu verabreichenden Medium zeigt. Der nicht näher dargestellte Füllkopf ist so ausgeführt, daß vor dem Befüllen des Zylinderraums 1 durch die Zerstäuberdüse 6 bzw. deren Mikroöffnung 10 hindurch zunächst Luft abgesaugt wird. Erst dann erfolgt die eigentliche Befüllung mit dem zu verabreichenden Medium. Nach Entfernung des Füllkopfes schließt sich die Membrandichtung 4 selbsttätig, so daß der Zylinderraum 1 nach Befüllung frei von Lufteinschlüssen ist. Dementsprechend lang ist die Haltbarkeit des abgefüllten Mediums. Die genannte Vorrichtung wird befüllt geliefert. In diesem Zustand befindet sich der Kolben 7 in der in Fig. 1 dargestellten Ausgangsstellung. In dieser wird er durch einen Rastmechanismus gehalten, der zur Verabreichung durch Ausübung von Druck auf eine an dem der Zerstäuberdüse 6 abgewandten Ende des Kolbens 7 ausgebildeten Kolbenbetätigungsfläche 20 überwunden werden muß. Die Kolbenbetätigungsfläche 20 ist bei der dargestellten Ausführungsform Teil eines zur Zerstäuberdüse 6 hin offenen, sich koaxial zum Kolben 7 erstrekkenden Zylindertopfes 8, welcher innerhalb eines mit dem fingerartigen Applikator 3 verbundenen Führungszylinders 11 verschieblich geführt ist. Der Rastmechanismus wird durch einen am Zylinderkopf 8 ausgebildeten Vorsprung, nämlich Ringvorsprung 21 einerseits, und eine an der Innenseite des Führungszylinders 11 komplementär ausgebildete Vertiefung, nämlich Ringnut 12, 13 andererseits definiert. Die der Zerstäuberdüse 6 abgewandte Begrenzung der an der Innenseite des Führungszylinders 11 ausgebildeten Ringnut dient zugleich als ein das Herausziehen des Kolbens 7 aus dem Zylinderraum 1 des Applikators 3 verhindernder Anschlag 12. Dieser Anschlag 12 wird bei der dargestellten Ausführungsform durch Einziehen des freien Umfangsrandes des Führungszylinders 11 gebildet. Der Führungszylinder 11 ist mit dem Applikator 3 und der den Zylinderraum 1 begrenzenden Zylinderwand 30 einstückig ausgebildet. Das gleiche gilt für den Kolben 7 und dessen Betätigungsteil 8, 20. In Fig. 5 ist der vorgenannte Rastmechanismus noch etwas deutlicher dargestellt.

Die vorbeschriebene Ausführungsform dient zur sogenannten Einmal-Applikation.

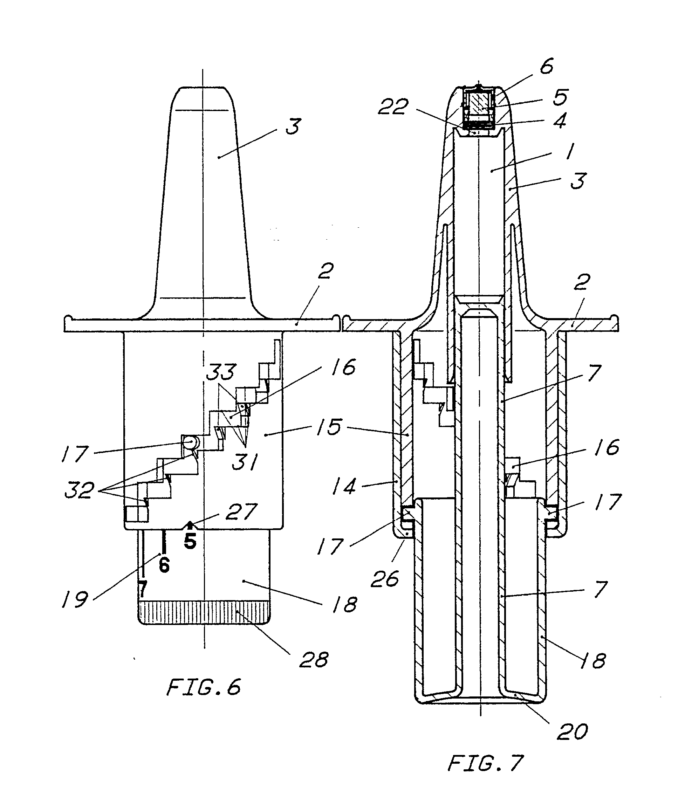

Die Fig. 6 und 7 beziehen sich auf eine Vorrichtung, die eine mehrmalige Applikation erlaubt. Dabei sind sämtliche Konstruktionsmerkmale, wie sie bereits anhand der Fig. 1 bis 5 beschrieben sind, in den Fig. 6 und 7 mit denselben Bezugsziffern versehen. Diesbezüglich kann auf die vorangehende Beschreibung dieser Merkmale Bezug genommen werden.

Die Vorrichtung nach den Fig. 6 und 7 zeichnet sich dadurch aus, daß der Kolben 7 schrittweise in den Zylinderraum 1 unter entsprechender Mehrfach-Verabreichung hineinbewegbar ist, wobei zu diesem Zweck ebenfalls an dem der Zerstäuberdüse 6 abgewandten Ende des Kolbens 7 eine Kolbenbetätigungsfläche 20 ausgebildet ist. Wie bei dem vorangegangenen Ausführungsbeispiel ist die Kolbenbetätigungsfläche 20 Teil eines zur Zerstäuberdüse 6 hin offenen, sich koaxial zum Kolben 7 erstreckenden Zylindertopfes 18, welcher innerhalb eines mit dem fingerartigen Applikator 3 verbundenen Führungszylinders 15 verschieblich geführt ist, wobei am Zylindertopf 18 zwei diametral angeordnete, sich jeweils radial erstreckende stift- bzw. zapfenartige Vorsprünge 17 ausgebildet sind, die jeweils in eine am Führungszylinder 15 ausgebildete Treppenführung 16 eingreifen. Durch Drehen und Verschieben des Kolbens 7 relativ zum Führungszylinder 15 ist jeweils eine Führungsstufe 31 überwindbar unter entsprechender Applikation einer durch die axiale Erstreckung der Führungsstufe vorbestimmten Dosis. Die Treppenführung 16 ist im vorliegenden Fall jeweils als Zylinderwand-Durchbruch ausgebildet. Über den Führungszylinder 15, der mit dem Applikator 3 einstückig verbunden ist, ist ein äußerer Abdeckzylinder geschoben, dessen der Zerstäuberdüse 6 abgewandter Umfangsrand 26 nach innen eingezogen ist unter Ausbildung eines Anschlags gegen unbeabsichtigtes Herausziehen des Kolbens 7 aus dem zugeordneten Zylinderraum 1.

Entsprechend Fig. 6 ist am der Zerstäuberdüse 6 abgewandten Umfangsrand 26 des äußeren Abdeckzylinders 14 eine Markierung, nämlich Markierungskerbe 27 ausgebildet, die mit einer Markierung, nämlich Strichmarkierung 19 an der Außenfläche des die Fingerbetätigungsfläche 20 umfassenden Zylindertopfes 18 korrespondiert, wobei der Umfangsabstand zwischen den letztgenannten Strichmarkierungen 19 der Umfangsteilung der am inneren Führungszylinder 15 ausgebildeten Treppenführung 16 bzw. dem jeweiligen Umfangsabstand zwischen benachbarten Führungsstufen 31 der vorgenannten Treppenführung 16 entspricht. Zum erleichterten Verdrehen des Bauteils 18 ist am freien und nach außen vorstehenden Umfangsrand desselben eine Rändelung 28 ausgebildet. Zum Zwecke der mehrmaligen Verabreichung einer vorbestimmten Dosis muß demnach das Bauteil 18 innerhalb einer Stufe 31 zunächst entgegen dem Uhrzeigersinn verdreht werden, bis die Zapfen 17 an den senkrechten Abschnitten der jeweiligen Stufe 31 anliegen. Dann kann der Kolben 7 in den Zylinderraum 1 geschoben werden unter entsprechender Verabreichung einer Medikamentendosis, und zwar bis die Zapfen 17 an der oberen Begrenzung der nächsten Stufe der Führungstreppe 16 anschlagen, usw.

Bei sämtlichen Ausführungsformen sind am Fuß bzw. an der Wurzel bzw. an dem der Zerstäuberdüse 6 abgewandten Ende des fingerartigen Applikators 3 radial vorstehende Stege oder ein radial vorstehender Flansch 2 ausgebildet, die bzw. der zur Abstützung von Zeige- und Mittelfinger des Benutzers bei Betätigung des Kolbens 7 unter Ausübung von Druck auf die dem Kolben 7 zugeordnete Kolbenbetätigungsfläche 20 mit dem Daumen dient. Der fingerartige Applikator 3 liegt dabei zwischen Zeige- und Mittelfinger.

Um eine Rückbewegung des Kolbens 7 durch den Benutzer zu verhindern, ist jeder Führungsstufe 31 vorzugsweise ein Sperrelement zugeordnet, welches nach Vorbeibewegen der Zapfen 17 eine Rückbewegung blockiert. Ein solches Sperrelement kann z. B. als Sperrkeil mit elastisch nachgiebiger Schrägfläche ausgebildet sein, über die der Zapfen 17 hinwegbewegt werden kann. Eine Rückbewegung ist durch eine sich senkrecht zur Bewegungsrichtung der Zapfen 17 erstreckende Anschlagfläche des Sperrkeils blockiert. Die vorgenannte Schrägfläche ist in Fig. 6 mit der Bezugsziffer 32 und die vorgenannte Anschlagfläche mit der Bezugsziffer 33 gekennzeichnet. Der durch die Flächen 32, 33 definierte Sperrkeil befindet sich jeweils im sich axial erstreckenden Führungsabschnitt der Führungsstufen 31, und zwar so, daß die Anschlagfläche auf Höhe der unteren Begrenzung einer jeden Führungsstufe 31 liegt.

Als Sperrelemente können auch blattfederartige Sperrzungen oder dgl. dienen. Vorzugsweise sind die Sperrelemente jedoch jeweils derart wirksam, daß sie eine axiale Rückbewegung des Kolbens verhindern. Eine Rückdrehung des Kolbens innerhalb einer Treppen- bzw. Führungsstufe ist unkritisch.

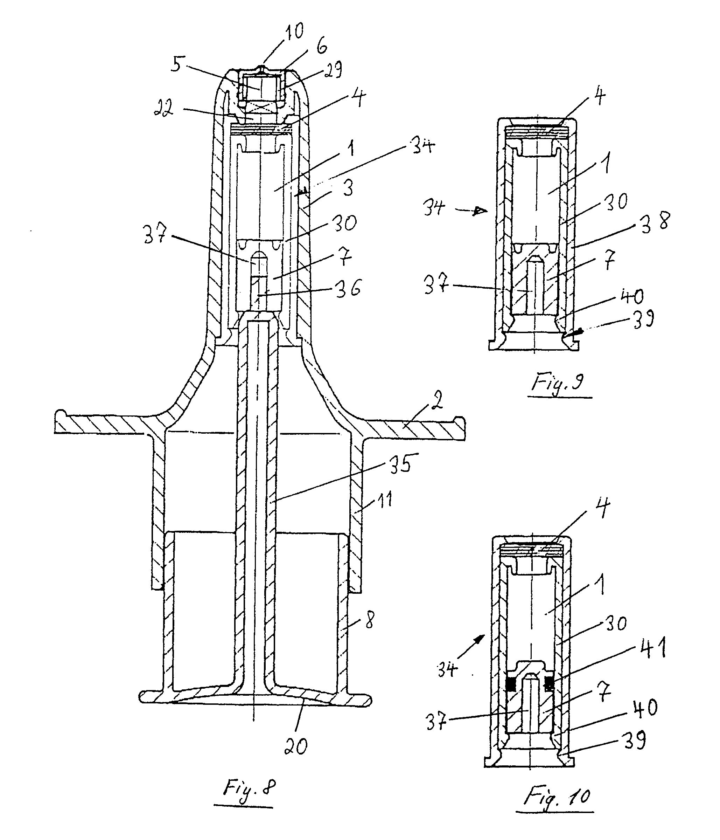

In den Fig. 8 bis 10 ist eine dritte Ausführungsform einer erfindungsgemäßen Vorrichtung dargestellt, wobei auch hier sämtliche Konstruktionsmerkmale, die bereits anhand der vorangegangenen Figuren beschrieben worden sind, in den Fig. 8 bis 10 mit denselben Bezugsziffern versehen sind. Diesbezüglich wird auf die vorangehende Beschreibung dieser Merkmale Bezug genommen.

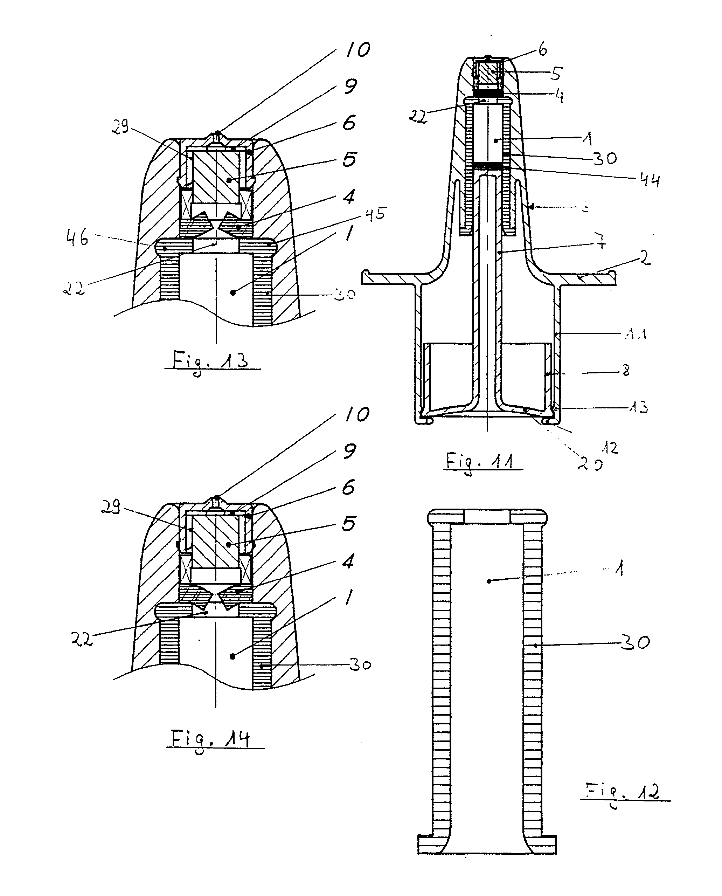

Die Vorrichtung nach den Fig. 8 bis 10 zeichnet sich dadurch aus, daß der Zylinderraum 1 durch eine gesonderte Patrone definiert ist, die vor dem Gebrauch der Vorrichtung im Applikator 3 positionierbar ist, so wie dies in Fig. 8 dargestellt ist. Die vorgenannte, den Zylinderraum 1 definierende Patrone 34 ist an der der Zerstäuberdüse 6 zugewandten Seite durch die membranartige Dichtung 4 und an der gegenüberliegenden Seite durch den Kolben 7 begrenzt. Der Kolben 7 ist Teil der Patrone 34, d. h. vom Kolben-Betätigungselement getrennt. Das Kolben-Betätigungselement ist durch einen Stößel 35 definiert, an dessen dem Kolben 7 abgewandten Ende die Kolbenbetätigungsfläche 20 ausgebildet ist. Die Verbindung zwischen Kolben 7 und Stößel 35 erfolgt durch einen Zapfen 36 am kolbenseitigen Ende des Stößels 35, welcher in eine entsprechende Axialbohrung 37 des Kolbens 7 einführbar ist (s. Fig. 8). Entsprechend den Fig. 9 und 10 umfaßt die Patrone 34 eine innere Zylinderhülse 30, die den Zylinderraum 1 seitlich begrenzt und innerhalb der der Kolben 7 verschieblich gelagert ist, sowie eine äußere Zylinderhülse 38, die über die innere Zylinderhülse 30 geschoben bzw. gestülpt ist unter Fixierung der Membrandichtung 4 zwischen dem zerstäuberdüsenseitigen Umfangsrand der inneren Zylinderhülse 30 und dem zerstäuberdüsenseitigen Umfangsrand der äußeren Zylinderhülse 38. Die beiden Zylinderhülsen 30, 38 sind im montierten Zustand miteinander verrastet (Rastvorsprung 39). Am der Zerstäuberdüse abgewandten Umfangsrand der inneren Zylinderhülse 30 ist ein sich radial nach innen erstreckender Ringvorsprung 40 ausgebildet, der ein Herausrutschen des Kolbens 7 aus der inneren Zylinderhülse 30 verhindert.

Die Patrone 34 gemäß Fig. 10 unterscheidet sich von derjenigen gemäß Fig. 9 nur dadurch, daß der Kolben 7 mit einer Kolben-Ringdichtung 41 versehen ist, die mit der Innenseite der inneren Zylinderhülse 30 korrespondiert. Die Dichtung 41 ist vorzugsweise als O-Ring ausgebildet und besteht aus Gummi oder dgl. Werkstoff.

Der Vorteil der Ausführungsform nach den Fig. 8 bis 10 besteht darin, daß der Betätigungsmechanismus des Applikators mehrfach verwendet werden kann. Es braucht lediglich die Patrone 34 ausgetauscht zu werden, d. h. die entleerte Patrone durch eine mit Wirkstoff gefüllte Patrone. Zu diesem Zweck wird lediglich das Kolben-Betätigungselement 8, 20, 35 aus dem Führungszylinder 11 herausgezogen, und zwar unter Mitnahme der Patrone 34. Anschließend wird der Stößel 35 aus der korrespondierenden Kolbenbohrung 37 herausgezogen. Anschließend wird in den Applikator 3 eine neue Patrone 34 eingesetzt und mit dem Stößel 35 über den Zapfen 36 verbunden. Die entsprechende Zapfenverbindung ist nicht unbedingt erforderlich, jedoch aus vorgenanntem Grund, d.h. zum Zwecke des Patronen-Austausches vorteilhaft bzw. zweckmäßig.

Im übrigen ist die Funktion des vorbeschriebenen Applikators vergleichbar mit der Funktion des Applikators nach den Fig. 1 bis 5 bzw. 6 und 7.

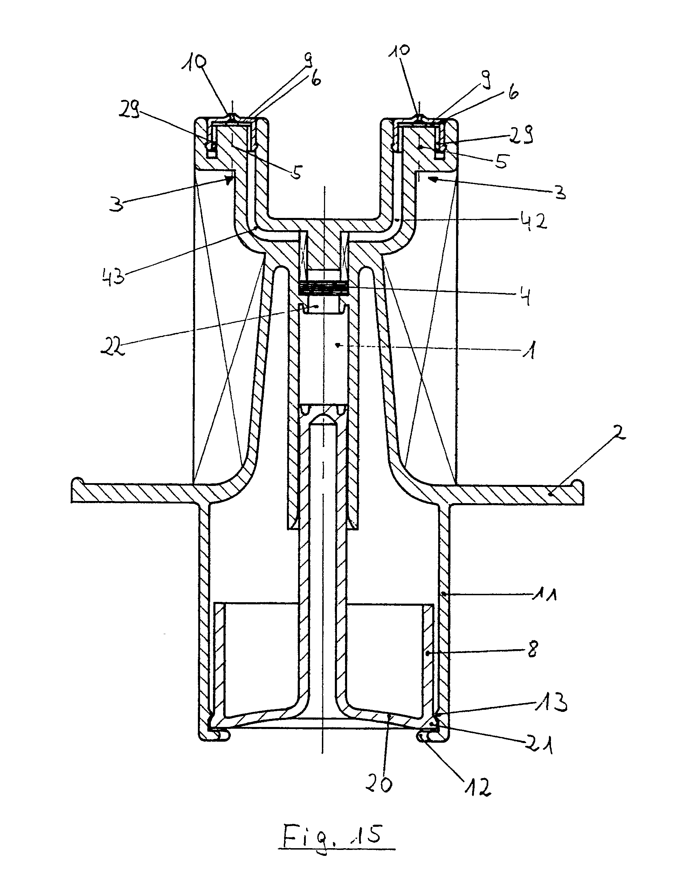

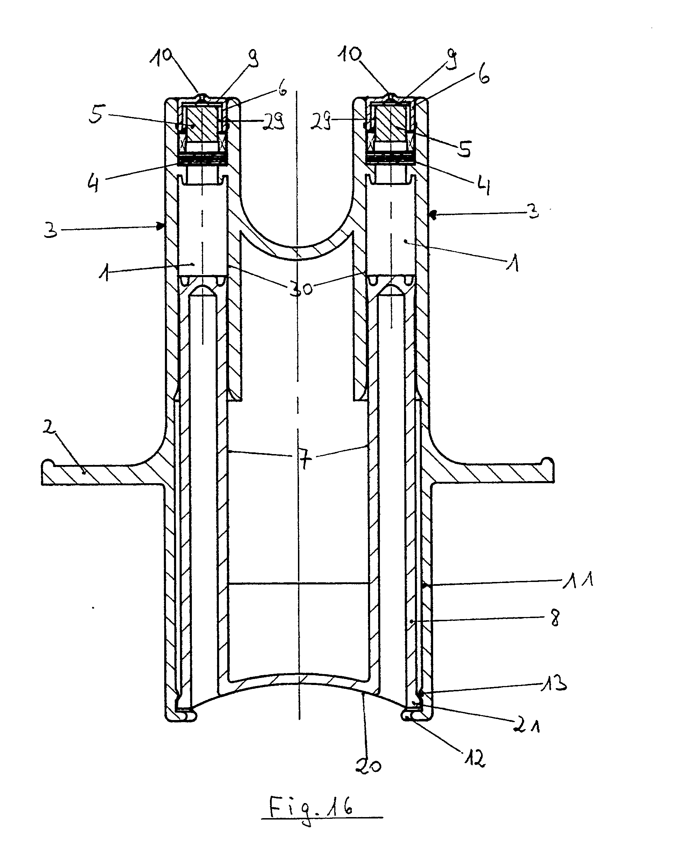

Die Ausführungsform nach den Fig. 11 bis 14 unterscheidet sich von derjenigen nach den Fig. 1 bis 4 nur dadurch, daß der Zylinderraum 1 durch einen gesonderten Einsatzzylinder 30 aus Glas oder dgl. impermeablem Material begrenzt ist, wobei dieser Einsatzzylinder innerhalb des Applikatorfingers mit Rastsitz (Rastnut 45/Rastvorsprung 46) fixiert ist, und zwar unter gleichzeitiger Klemmfixierung der membranartigen Dichtung 4.

Desweiteren zeichnet sich die vorbeschriebene Ausführungsform dadurch aus, daß an der Kolbenfläche des Kolbens 7 eine scheibenförmige Kolbendichtung 44 anliegt, und zwar nur lose aufliegt. Die Kolbendichtung 44 ist also am Kolben 7 nicht fixiert. Trotzdem ist die Kolbendichtung voll funktionsfähig, da sie beim Befüllen des Zylinderraums 1 zusammen mit dem Kolben 7 in Richtung aus dem Zylinderraum 1 herausgeschoben wird. Umgekehrt wird sie bei Betätigung des Kolbens 7 mit diesem mitbewegt. Bedingt durch die innerhalb des Zylinderraums 1 sowohl bei der Befüllung als auch bei der Applikation herrschenden Überdrücke wird also die Kolbendichtung 44 stets in Anlage an der Kolbenfläche des Kolbens 7 gehalten.

Die Ausführungsformen nach Fig. 15 und 16 zeichnen sich dadurch aus, daß sie jeweils zwei, gemeinsam betätigbare Applikatoren 3 umfassen, die so voneinander beabstandet sind, daß das zu verabreichende Medium gleichzeitig in beide Nasenlöcher eines Benutzers einsprühbar ist. Bei der Ausführungsform nach Fig. 16 ist jedem einzelnen Applikator 3 eine gesonderte Kolben-Zylinder-Einheit der vorbeschriebenen Art, nämlich hier der anhand der Fig. 1 bis 5 beschriebenen Art, zugeordnet, wobei beide Kolben 7 über eine gemeinsame Kolbenbetätigungsfläche 20 miteinander verbunden sind. Dementsprechend sind die beiden Kolben 7 gemeinsam betätigbar. Durch beide Zerstäuberdüsen 6 wird also die gleiche Menge Heilmedium verabreicht.

Die Ausführungsform nach Fig. 15 ist dadurch gekennzeichnet, daß beiden Applikatoren 3 eine gemeinsame Kolben-Zylinder-Einheit der vorbeschriebenen Art zugeordnet ist, wobei der Zylinderraum 1 über zwei, jeweils hinter der membranartigen Dichtung 4 ausgebildete Fluidkanäle 42 und 43 mit den Zerstäuberdüsen 6 der beiden Applikatoren 3 fluidverbunden ist. Hinsichtlich der Funktionsweise entspricht daher die Ausführungsform nach Fig. 15 derjenigen nach Fig. 16. Lediglich der Zylinderinhalt ist bei beiden Ausführungsformen unterschiedllich.

Sämtliche in den Anmeldungsunterlagen offenbarten Merkmale werden als erfindungswesentliche beansprucht, soweit sie einzeln oder in Kombination gegenüber dem Stand der Technik neu sind.

## Patentansprüche

1. Vorrichtung zur transnasalen oder oralen Verabreichung von Medikamenten oder dgl. Medien, mit einem fingerartigen Applikator (3), an dessen freiem Ende eine Zerstäuberdüse (6) angeordnet ist und der einen Zylinderraum (1) zur Aufnahme des zu verabreichenden Mediums umfaßt, wobei dieses mittels eines Kolbens (7) aus dem Zylinderraum (1) über die Zerstäuberdüse (6) verabreichbar ist,
**dadurch gekennzeichnet, daß**
der Zerstäuberdüse (6) eine membranartige Dichtung (4) mit einem sich nur unter Druck öffnenden Fluiddurchgang vorgeordnet ist.

2. Vorrichtung nach Anspruch 1,
**dadurch gekennzeichnet, daß** die Membrandichtung (4) in einem Durchgang (22) zwischen Zylinderraum (1) und Zerstäuberdüse (6) angeordnet ist, insbesondere unter Klemmfixierung längs ihres Umfangsrandes.

3. Vorrichtung nach Anspruch 1 oder 2,
**dadurch gekennzeichnet, daß** zur Ausbildung des Fluiddurchgangs die Membrandichtung (4) einen geraden (23), sternförmigen (24) oder kreuzförmigen (25) Schlitz aufweist.

4. Vorrichtung nach einem der Ansprüche 1 bis 3,
**dadurch gekennzeichnet, daß** die Klemmfixierung der Membrandichtung (4) durch ein zwischen dieser und der Zerstäuberdüse (6) angeordnetes, gleichzeitig zur Verteilung des zu verabreichenden Mediums vor dessen Austritt durch die Zerstäuberdüse dienendes Zwischenstück (5) erfolgt, wobei an der der Zerstäuberdüse (6) zugewandten Seite des Zwischenstücks (5) eine Einrichtung (9) zur Verwirbelung des durch die Zerstäuberdüse (6) austretenden Mediums angeordnet ist.

5. Vorrichtung nach einem der Ansprüche 1 bis 4,
**dadurch gekennzeichnet, daß** der Kolben (7) in Ausgangsstellung durch einen Rastmechanismus gehalten ist, der zur Applikation durch Ausübung von Druck auf eine an dem der Zerstäuberdüse (6) abgewandten Ende des Kolbens (7) ausgebildete Kolbenbetätigungsfläche (20) überwunden werden muß.

6. Vorrichtung nach Anspruch 5,
**dadurch gekennzeichnet, daß** die Kolbenbetätigungsfläche (20) Teil eines zur Zerstäuberdüse (6) hin offenen, sich koaxial zum Kolben (2) erstreckenden Zylindertopfes (8) ist, welcher innerhalb eines mit dem fingerartigen Applikator (3) verbundenen Führungszylinders (11) verschieblich geführt ist.

7. Vorrichtung nach Anspruch 6,
**dadurch gekennzeichnet, daß** der Rastmechanismus durch einen am Zylindertopf (8) ausgebildeten Vorsprung, insbesondere Ringvorsprung (21) einerseits und eine an der Innenseite des Führungszylinders (11) komplementär ausgebildete Vertiefung, insbesondere Ringnut (12, 13) andererseits definiert ist.

8. Vorrichtung nach Anspruch 7,
dadurch gekennzeichnet, daß die der Zerstäuberdüse (6) abgewandte Begrenzung der an der Innenseite des Führungszylinders (11) ausgebildeten Ringnut zugleich als ein das Herausziehen des Kolbens (7) aus dem Zylinderraum (1) des Applikators (3) verhindernder Anschlag (12) dient.

9. Vorrichtung nach einem der Ansprüche 1 bis 4,
dadurch gekennzeichnet, daß der Kolben (7) schrittweise in den Zylinderraum (1) unter entsprechender Mehrfach-Applikation hineinbewegbar ist, wobei zu diesem Zweck an dem der Zerstäuberdüse (6) abgewandten Ende des Kolbens (7) eine Kolbenbetätigungsfläche (20) ausgebildet ist.

10. Vorrichtung nach Anspruch 9,
dadurch gekennzeichnet, daß die Kolbenbetätigungsfläche (20) Teil eines zur Zerstäuberdüse (6) hin offenen, sich koaxial zum Kolben (7) erstreckenden Zylindertopfes (18) ist, welcher innerhalb eines mit dem fingerartigen Applikator (3) verbundenen Führungszylinders (15) verschieblich geführt ist, wobei am Zylindertopf (18) wenigstens ein, insbesondere zwei diametral angeordnete, sich jeweils radial erstreckende Vorsprünge (17) ausgebildet sind, die jeweils in eine am Führungszylinder (15) ausgebildete Treppenführung (16) eingreifen, so daß durch Drehen und Verschieben des Kolbens (7) relativ zum Führungszylinder (15) jeweils eine Führungsstufe (31) überwindbar ist unter entsprechender Applikation einer durch die axiale Höhe der Führungsstufe (31) vorbestimmten Dosis.

11. Vorrichtung nach Anspruch 10,
dadurch gekennzeichnet, daß die Treppenführung (16) jeweils als Zylinderwand-Durchbruch ausgebildet ist, und daß sich über den Führungszylinder (15) ein äußerer Abdeckzylinder (14) erstreckt, dessen der Zerstäuberdüse (6) abgewandte bzw. freie Umfangsrand (26) nach innen eingezogen ist unter Ausbildung eines Anschlags gegen unbeabsichtigtes Herausziehen des Kolbens (7) aus dem zugeordneten Zylinderraum (1).

12. Vorrichtung nach Anspruch 11,
dadurch gekennzeichnet, daß am der Zerstäuberdüse (6) abgewandten Umfangsrand (26) des äußeren Abdeckzylinders (14) eine Markierung, insbesondere Markierungskerbe (27) oder Markierungsvorsprung, ausgebildet ist, die mit einer Markierung, insbesondere Strichmarkierung (19), an der Außenfläche des die Fingerbetätigungsfläche (20) umfassenden Zylindertopfes (18) korrespondiert, wobei der Umfangsabstand zwischen den letztgenannten Markierungen (19) der Umfangsteilung der am inneren Führungszylinder (15) ausgebildeten Treppenführung (16) bzw. dem jeweiligen Umfangsabstand zwischen benachbarten Führungsstufen (31) der vorgenannten Treppenführung (16) entspricht.

13. Vorrichtung nach einem der Ansprüche 10 bis 12,
dadurch gekennzeichnet, daß am freien und nach außen vorstehenden Umfangsrand des Zylindertopfes (18) eine Rändelung (28) ausgebildet ist.

14. Vorrichtung nach einem der Ansprüche 1 bis 13,
dadurch gekennzeichnet, daß am Fuß bzw. am der Zerstäuberdüse (6) abgewandten Ende des fingerartigen Applikators (3) radial vorstehende Stege oder ein radial vorstehender Flansch (2) ausgebildet ist, die bzw. der zur Abstützung von Zeige- und Mittelfinger des Benutzers bei Betätigung des Kolbens (7) unter Ausübung von Druck auf die dem Kolben (7) zugeordnete Kolbenbetätigungsfläche (20) mit dem Daumen dient.

15. Vorrichtung nach einem der Ansprüche 9 bis 14,
dadurch gekennzeichnet, daß jeder Führungsstufe (31), insbesondere dem sich axial erstreckenden Abschnitt jeder Führungsstufe (31) ein Sperrelement (32, 33) zugeordnet ist, welches eine Applikationsbewegung des Kolbens (7) erlaubt, eine Rückbewegung desselben dagegen blockiert.

16. Vorrichtung nach einem der Ansprüche 1 bis 15,
dadurch gekennzeichnet, daß der Zylinderraum (1) für die Aufnahme des zu verabreichenden Mediums durch eine im Applikator (3) positionierbare Patrone (34) definiert ist, die an der der Zerstäuberdüse (6) zugewandten Seite durch die membranartige Dichtung (4) und an der gegenüberliegenden Seite durch den Kolben (7) begrenzt ist.

17. Vorrichtung nach Anspruch 16,

**dadurch gekennzeichnet,** daß der Kolben (7) durch einen Stößel (35) in den Zylinderraum (1) hinein verschiebbar ist, wobei am dem Kolben (7) abgewandten Ende des Stößels (35) die Kolbenbetätigungsfläche (20) ausgebildet ist.

18. Vorrichtung nach einem der Ansprüche 1 bis 17,
   **dadurch gekennzeichnet,** daß der Zylinderraum (1) durch einen gesonderten Zylinder aus impermeablem Material, insbesondere Glas, begrenzt ist.

19. Vorrichtung nach einem der Ansprüche 1 bis 18,
   **dadurch gekennzeichnet,** daß an der Kolbenfläche des Kolbens (7) eine scheibenförmige Kolbendichtung (44) anliegt, und zwar vorzugsweise nur lose aufliegt.

20. Vorrichtung zur transnasalen Verabreichung von Medikamenten oder dergleichen Medien,
   **dadurch gekennzeichnet,** daß sie zwei gemeinsam betätigbare Applikatoren (3) nach einem oder mehreren der Ansprüche 1 bis 19 umfaßt, die so voneinander beabstandet sind, daß das zu verabreichende Medium gleichzeitig in beide Nasenlöcher einsprühbar ist.

21. Vorrichtung nach Anspruch 20,
   **dadurch gekennzeichnet,** daß jedem einzelnen Applikator (3) eine gesondere Kolben(7)-Zylinder(1)-Einheit zugeordnet ist, wobei beide Kolben (7) über eine gemeinsame Kolbenbetätigungsfläche (20) miteinander verbunden sind (Fig. 16).

22. Vorrichtung nach Anspruch 20,
   **dadurch gekennzeichnet,** daß beide Applikatoren (3) eine gemeinsame Kolben(7)-Zylinder-(1)-Einheit aufweisen, wobei der Zylinderraum (1) über zwei, jeweils hinter der membranartigen Dichtung (4) ausgebildete Fluidkanäle (42, 43) mit den Zerstäuberdüsen (6) der beiden Applikatoren (3) fluidverbunden ist.

FIG.2

FIG.1

FIG.3

FIG.5

FIG.4

FIG.6

FIG.7

Fig. 8

Fig. 9

Fig. 10

11

Fig. 13

Fig. 14

Fig. 11

Fig. 12

Fig. 15

Fig.16

**Europäisches Patentamt**

# EUROPÄISCHER RECHERCHENBERICHT

Nummer der Anmeldung

## EP 91 10 5082

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,A | FR-A-2 625 981 (VALOIS)<br>* Seite 1, Zeile 8 - Zeile 17 * * Seite 2, Zeile 24 - Seite 4, Zeile 28; Abbildungen 2-6 *<br>— — — | 1-6,9,14,<br>19,10,15,<br>18 | B 05 B 11/02<br>A 61 M 11/00 |
| Y | FR-A-2 635 084 (SOFAB)<br>* Seite 1, Zeile 39 - Seite 2, Zeile 12 * * Zusammenfassung; Abbildungen 1,4,5 *<br>— — — | 1-6,9,14,<br>19 | |
| A | US-A-4 067 499 (COHEN, M.J)<br>* Zusammenfassung; Ansprüche 1,2; Abbildungen 3-6 *<br>— — — — — | 1 | |

| RECHERCHIERTE SACHGEBIETE (Int. Cl.5) |
|---|
| A 61 M<br>B 05 B |

Der vorliegende Recherchenbericht wurde für alle Patentansprüche erstellt

| Recherchenort | Abschlußdatum der Recherche | Prüfer |
|---|---|---|
| Den Haag | 19 Juli 91 | GIMENEZ BURGOS R. |